# EUROPÄISCHE PATENTANMELDUNG

(11) **EP 0 705 901 A2**
(43) Veröffentlichungstag der Anmeldung: **10.04.1996**
(21) Anmeldenummer: 95115082.0
(22) Anmeldetag: 25.09.1995
(51) Int. Cl.: C12N 9/64, A61K 38/48

(54) **Verfahren zur Trennung von rekombinantem pro-Faktor IX von rekombinantem Faktor IX**

(30) Priorität: 04.10.1994 DE 4435520
(71) Anmelder: IMMUNO Aktiengesellschaft, A-1221 Wien (AT)
(72) Erfinder: Fischer, Bernhard, Dr., A-1120 Wien (AT); Mitterer, Artur, Dr., A-2304 Orth/Donau (AT); Dorner, Friedrich, Prof. Dr., A-1230 Wien (AT); Eibl, Johann, Dr., A-1180 Wien (AT)
(74) Vertreter: Kolb, Helga, Dr. Dipl.-Chem.

(57) **Zusammenfassung**

Es wird ein Verfahren zur Trennung von rekombinantem pro-Faktor IX von rekombinantem Faktor IX durch eine chromatographische Methode beschrieben. Nach einer bevorzugten Ausführungsform wird die Mischung der beiden Proteine an einen Ionenaustauscher gebunden und pro-Faktor IX und Faktor IX werden durch Pufferlösungen mit unterschiedlichen Salzkonzentrationen und/oder pH-Werten voneinander getrennt eluiert.

Nach dem erfindungsgemäßen Verfahren sind die Faktoren pro-Faktor IX und Faktor IX in hochreiner Form und jeweils frei vom anderen Faktor erhältlich.

## Beschreibung

Die Erfindung betrifft ein Verfahren zur Trennung von rekombinantem pro-Faktor IX von rekombinantem Faktor IX.

Bei der Blutgerinnung erfolgt die Umwandlung des flüssigen Blutes in den Blutkuchen, eine gallertige Masse, die die Abdichtung verletzter Blutgefäße durch Pfropfbildung bewirkt. Dabei erfolgt die Umwandlung des im Plasma vorhandenen löslichen Fibrinogens in den faserig-gallertigen Gerinnungsstoff, das Fibrin, in einem mehrstufigen Prozeß (sogenannte Blutgerinnungs-Kaskade), an dem mindestens 15 verschiedene, mit römischen Ziffern gekennzeichnete Blutgerinnungs-Faktoren beteiligt sind, von denen jeder, wenn er aktiviert ist, die jeweils nächste inaktive Stufe aktiviert.

Von den Gerinnungsfaktoren zirkulieren Calicum-Ionen (Faktor IV), Fibrinogen (Faktor I) und Prothrombin (Faktor II) ständig im Blut, andere werden durch Gewebeverletzung (Faktor III) oder Kontakt mit Kollagen oder Phosphorlipiden aus Thrombocyten (Faktor XII) aktiviert. Unter den übrigen Blutgerinnungs-Faktoren befinden sich mehrere Serin-Proteasen, wie Kallikrein, Thrombin und die aktivierten Faktoren VII, IX, X und XI.

Die Thrombocyten setzen sich unter Anwesenheit von von Willebrand-Faktor (ein Bestandteil des Gerinnungs-Faktors VIII) an Kollagen des verletzten Bindegewebs durch Adhäsion fest. Sie verändern ihre Form und bilden Fortsätze aus, und zudem erleichtert ihre äußere Membran die Adhäsion weiterer Thrombocyten. Danach setzen sie verschiedene Substanzen aus ihren Granula frei, wodurch eine Gefäßkonstriktion sowie die Anlagerung und die Aktivierung anderer Faktoren der plasmatischen Blutgerinnung bewirkt werden.

Bei der Hämophilie (Bluterkrankheit) ist die Blutgerinnung durch Mangel an bestimmten plasmatischen Blutgerinnungs-Faktoren gestört. Bei der Hämophilie A beruht die Blutungsneigung auf einem Mangel an Faktor VIII, bei der Hämophilie B auf einem Mangel an Faktor IX. Es kann dabei entweder die Synthese des Faktor-Proteins vermindert sein oder es wird ein defektes Molekül mit herabgesetzter Aktivität gebildet. Die Behandlung der Hämophilie erfolgt durch Ersatz des fehlenden Gerinnungsfaktors durch Faktorenkonzentrate aus Blutkonserven.

Mehrere der an der Blutgerinnung des Menschen beteiligten Proteine besitzen eine Affinität zu Metallionen, wie z.B. Ca2+-Ionen. Diese Affinität ist für die Funktion der Gerinnungsfaktoren absolut notwendig. Die Bindung erfolgt über Glutaminsäurereste; dabei werden in einer Vitamin K-abhängigen Reaktion mehrere Glutaminsäurereste (Glu) der N-terminalen Gla-Region verschiedener Gerinnungsfaktoren in 4-Carboxy-L-Glutaminsäure (Gla) umgewandelt (s. A. Tulinsky, Thromb. Haemost. 66 (1991) 16-31). Diese Gla-Reste bedingen dann die Bindung von zweiwertigen Metallionen (s. B. Furie und B.C. Furie, Cell 53 (1988) 508-518).

Bei der Biosynthese der Vitamin K-abhängigen Gerinnungsfaktoren im Menschen wird zuerst ein Vorläufermolekül gebildet, welches N-terminal eine zusätzliche pre-pro-Sequenz aufweist. Die pre-Sequenz stellt eine Signalsequenz dar, die den orientierten Transport des Proteins in der Zelle bedingt. Beim Ausschleusen des Proteins aus der Zelle wird diese pre-Sequenz abgespalten. Die pro-Sequenz besteht aus ca. 15 bis 18 Aminosäuren und dient als Erkennungsmuster bei der Umwandlung der Glutaminsäurereste in die 4-Carboxy-L-Glutaminsäure. Nach erfolgter Carboxylierung wird auch die pro-Sequenz abgespalten. Wird die pro-Sequenz nicht oder nur unvollständig abgespalten, so entstehen nur gering aktive Gerinnungsfaktoren. Humaner Faktor IX hat ein Molekulargewicht von ca. 55000 Dalton. Seine pro-Sequenz besteht aus 18 Aminosäuren, wodurch sich das Molekulargewicht um ca. 2000 Dalton erhöht. Bei der Reinigung von Faktor IX aus Plasma erhält man fast ausschließlich aktiven Faktor IX. Die Reinigung von Faktor IX aus Plasma ist allerdings sehr schwierig, da der Faktor IX nur in sehr geringer Konzentration im Plasma vorhanden ist (5 µg/ml; s. L.O. Andersson, Thrombosis Research 7 (1975) 451-459).

Es ist deshalb wünschenswert, rekombinanten Faktor IX für die Behandlung von an Hämophilie B erkrankten Patienten verfügbar zu haben.

Die zur Expression verwendete DNA-Sequenz des Faktors IX enthält auch die pre-pro-Sequenzen. Von den exprimierenden Zellsystemen wird erwartet, daß sie diese Sequenzen zur vollständigen Prozessierung des Faktors IX quantitativ abspalten und einen physiologisch aktiven Gerinnungsfaktor sezernieren. Es wurde doch festgestellt, daß im Falle des Faktors IX das zelleigene Potential transformierter Zellen zur Abspaltung der pro-Sequenz nicht ausreicht. Verschiedene Ansätze zur Erzeugung von rekombinantem Faktor IX führten daher zu Produkten mit nur geringer Aktivität (R.J. Kaufman et al, J. Biol. Chem 261 (1986) 9622-9628; S.Busby et al., Nature 316 (1985) 271-273; D.J.G. Rees et al., EMBO J. 7 (1988) 2053-2061). Dies kann auf eine unvollständige Abspaltung der pro-Sequenz zurückzuführen sein (P. Meulien et al., Prot. Engineer. 3 (1990) 629-633), weil in den Zellüberständen eine Mischung aus rekombinant produziertem pro-Faktor IX und Faktor IX vorhanden ist.

Eine Verbesserung der Gewinnung von rekombinantem, physiologisch aktivem Faktor IX konnte bisher nur durch gentechnische Manipulation der pro-Sequenz erzielt werden. So wurde beispielsweise versucht, die pro-Sequenz von Faktor VII an die DNA-Sequenz von Faktor IX zu koppeln, um ein effektiveres Abspalten der pro-Sequenz zu erreichen (K. Berkner et al., Current Advances in Vitamin K Research, Elsevier Science Publishing Co. Inc. (1988) 199-207). P. Meulien et al. (Prot. Engineer. 3 (1990) 629-633) untersuchten den Einfluß von Mutationen im Bereich der Propeptidspaltstelle von Faktor IX. Sie stellten fest, daß durch Einführen einer Punktmutation an der Position +1 (Alanin gegen Thyrosin) die Ausbeute an aktivem Faktor IX deutlich erhöht werden kann; im Vergleich mit einem Wildtyp-Faktor IX, der eine spezifische Aktivität von 45-55 % nach einer Reinigung über eine DEAE-Spherodex-Säule und stufenweiser Elution mit 0,3 M NaCl im physiologischen pH-Bereich zeigt, wurde für den mutierten Faktor IX eine spezifische Aktivität von 85 bis 100 % gefunden.

Aufgabe der vorliegenden Erfindung ist es, ein Verfahren bereitzustellen, mit dem auf effiziente, einfache und sichere Weise rekombinanter inaktiver pro-Faktor IX von rekombinantem aktivem Faktor IX getrennt werden kann.

Diese Aufgabenstellung wird mit dem Gegenstand der vorliegenden Erfindung gelöst.

Gegenstand der vorliegenden Erfindung ist ein Verfahren gemäß Patentanspruch 1 zur chromatographischen Trennung von rekombinantem pro-Faktor IX von rekombinantem Faktor IX. Diese chromatographische Trennung kann dadurch erfolgen, daß die Mischung der beiden Proteine an einen Ionenaustauscher bindet und pro-Faktor IX und Faktor IX durch Pufferlösungen mit unterschiedlichen Salzkonzentrationen und/oder pH-Werten voneinander getrennt eluiert werden. Die chromatographische Trennung kann aber auch dadurch erfolgen, daß eine chromatographische Säule verwendet wird, an der eines der Proteine quantitativ gebunden wird und das andere Protein im Eluat aufgefangen wird. Das gebundene Protein kann dann durch Veränderung der Pufferzusammensetzung wieder eluiert werden.

Bevorzugte Ausgestaltungen dieses Verfahrens sind Gegenstand der Ansprüche 2 bis 18.

Weiterer Gegenstand der vorliegenden Erfindung ist ein hoch reiner rekombinanter Faktor IX, der frei ist von pro-Faktor IX, gemäß Patentanspruch 19 oder 20, der nach dem erfindungsgemäßen Verfahren erhältlich ist.

In einer bevorzugten Ausführungsform beträgt das Verhältnis von Faktor IX-Antigen zu aktivem Faktor IX ≦ 1,1.

Weiterer Gegenstand ist auch ein hochreiner rekombinanter pro-Faktor IX, der frei ist von Faktor IX und therapeutisch als Antagonist zu Faktor IX eingesetzt werden kann, gemäß Patentanspruch 22 oder 23, oder nach dem erfindungsgemäßen Verfahren erhältlich ist.

Gegenstand der vorliegenden Erfindung ist ferner eine pharmazeutische Zusammensetzung gemäß Anspruch 24, die dadurch gekennzeichnet ist, daß sie den erfindungsgemäßen hochreinen Faktor IX in einem physiologisch annehmbaren Träger enthält, sowie eine pharmazeutische Zusammensetzung gemäß Anspruch 25, die dadurch gekennzeichnet ist, daß sie einen erfindungsgemäßen hochreinen pro-Faktor IX in einem physiologisch annehmbaren Träger enthält.

Rekombinanter Faktor IX wurde gemäß dafür üblicher Verfahren nach Infektion von Verozellen (Affen-Nierenzellen) mit Vaccinia-Virus in Zellkulturtechnik gewonnen. Die Vero/Vaccinia-Expressionssysteme und Zellkulturbedingungen werden in F.G. Falkner et al., Thrombosis and Haemostasis 68 (1992) 119-124, N.Barrett et al., AIDS Res. 5 (1989) 159-171 und F. Dorner et al., AIDS Vaccine Research and Clinical Trials, Marcel Dekker, Inc. New York (1990) ausführlich beschrieben. Die Expression von rekombinantem Faktor IX erfolgt in synthetischem DMEM-Standardmedium (Dulbeccos minimal essential medium). Der Kulturüberstand wurde durch Zentrifugation abgetrennt.

Die Reinigung von rekombinantem Faktor IX aus dem zellfreien Kulturmedium erfolgte durch übliche Verfahren, wie sie z.B. von L.O. Andersson (l.c.) beschrieben werden. Als Ergebnis wird eine Mischung aus rekombinatem Faktor IX und pro-Faktor IX erhalten.

Für das erfindungsgemäße Verfahren können als Ionenaustauscher natürliche und synthetische hydrophile Gele verwendet werden. Vorzugsweise werden Gele mit stark anionischen Austauschgruppen verwendet, z.B. als Vertreter der natürlichen Gele QAE (QAE-SephadexR, ein stark basischer Anionen-Austauscher aus Dextran-Gelen, die durch Einführung von N,N-Diethyl-N-(2-hydroxy-1-propyl)-ammonio-ethyl-Gruppen modifiziert sind), DEAE (DEAE-Cellulose, Diethylaminoethyl-Cellulose) oder TMAE (TMAE-Cellulose, Trimethylammonioethyl-Cellulose).

Für das erfindungsgemäße Verfahren ist aber auch eine Immunaffinitätschromatographie geeignet, wobei Antikörper, die entweder das Pro-Peptid von pro-Faktor IX oder selektiv Faktor IX binden, an einer geeigneten Matrix immobilisiert werden. Dabei sind monoklonale und polyklonale Antikörper gleichermaßen geeignet.

Die Elution kann mittels Pufferlösungen mit unterschiedlichen Salzkonzentrationen oder mit underschiedlichen pH-Werten, oder einer Kombination beider Pufferlösungen, erfolgen.

Als Elutionslösungen mit unterschiedlichen pH-Werten werden vorzugsweise solche mit pH-Werten zwischen 5,0 und 10,0 verwendet. Die Elution mittels Pufferlösungen mit unterschiedlichen Salzkonzentrationen erfolgt vorzugsweise mit Pufferlösungen, die ein Salz eines univalenten Kations enthalten, wie insbesondere NaCl. Die Salzkonzentration liegt vorzugsweise im Bereich von 10 bis 1000 mM, und insbesondere im Bereich von 150 bis 400 mM.

Die Elution des gebundenen Proteins von der Immunaffinitätssäule durch eine Veränderung in der Zusammensetzung der Pufferlösung kann durch Zugabe eines anorganischen oder organischen Salzes geschehen, wobei vorzugsweise NaCl, MgCl2, KSCN oder Harnstoff verwendet werden, oder es wird ein hydrophobes Agens, wie zum Beispiel Ethylenglykol zugegeben. Die Salzkonzentration liegt vorzugsweise in einem Bereich von 500 mM bis 3,5 M, insbesondere in einem Bereich von 1,0 bis 3,5 M. Die Agenskonzentration liegt vorzugsweise in einem Bereich von 0,1 bis 50 Gew.-%, vorzugsweise 0.1 - 10 Gew.-%. Die Veränderung der Pufferzusammensetzung kann aber auch durch die Veränderung des pH-Wertes des Puffers erfolgen, wobei ein Senken des pH-Wertes unter pH 7,0, vorzugsweise unter pH 5,0, beziehungsweise ein Erhöhen des pH-Wertes über 8,0, vorzugsweise über 9,0 möglich ist.

Im erfindunsgemäßen Verfahren erfolgt die Elution von pro-Faktor IX und Faktor IX vorzugsweise durch einen steigenden Salzgradienten und/oder einen fallenden pH-Gradienten.

Die Elution des pro-Faktors IX erfolgt dabei bei geringeren Salzkonzentrationen oder höheren pH-Werten als die Elution des Faktors IX.

Das nach dem erfindungsgemäßen Verfahren erhältliche Verhältnis von Faktor IX-Antigen zu aktivem Faktor IX beträgt vorzugsweise ≦ 1,1.

Nach dem erfindungsgemäßen Verfahren läßt sich auf effiziente und einfache Weise ein rekombinanter Faktor IX, der frei ist von pro-Faktor IX, mindestens jedoch 95 % im besonderen 98 % rein ist, erhalten. Ebenso kann mit diesem Verfahren ein rekombinanter pro-Faktor IX erhalten werden, der frei ist von Faktor IX, mindestens jedoch 95 % im besonderen 98 % rein ist. Dieser pro-Faktor IX wird therapeutisch als Antagonist zu Faktor IX eingesetzt.

Zur Herstellung pharmazeutischer Präparationen werden die getrennten hochreinen Faktor IX und pro-Faktor IX enthaltenden Fraktionen vorzugsweise aufkonzentriert und als Konzentrat weiter verarbeitet.

Die Herstellung der pharmazeutischen Zusammensetzungen kann auf an sich bekannte und übliche Weise erfolgen. Vorzugsweise werden die hochreinen Produkte (Faktor IX oder pro-Faktor IX) oder ihre Konzentrate mit einem geeigneten physiologisch verträglichen Träger vermischt. Als Träger dient vorzugsweise eine physiologische Kochsalzlösung.

Die pharmazeutischen Zusammensetzungen können in einer zur Behandlung von Hämophilie üblichen und gebräuchlichen Dareichungsform vorliegen; vorzugsweise liegen sie in Form eines zur Infusion geeigneten Präparates vor.

Die Verwendung von pro-Faktor IX basiert auf dessen identischer Bindungsfähigkeit zu Calciumionen und Phospholipiden, die durch die sowohl in Faktor IX als auch in pro-Faktor IX vorhandenen identischen Gla- und EGF-Regionen vermittelt wird. Die Bindung von pro-Faktor IX verhindert die Bindung von Faktor IX daher kompetitiv und setzt somit als Antagonist die physiologische Wirkung von Faktor IX herab.

Dies ist von großer Bedeutung, wenn aufgrund einer bestimmten medizinischen Indikation die Blutgerinnungskaskade selektiv blockiert werden muß. Herkömmliche Antikoagulantien, wie Heparin oder Coumarin zeigen neben dem gewünschten Effekt starke Nebenwirkungen. Ihre kurze Halbwertszeit verlangt außerdem häufige Applikation. Seit kurzer Zeit wird auch der Einsatz von gentechnisch veränderten Blutgerinnnungsfaktoren, zum Beispiel Faktor VII und Faktor X, als Antikoagulantien diskutiert. Spezifisch für die Hemmung von Faktor IX wurden Peptide beschrieben, die die EGF-Domäne von Faktor IX kodieren und die Bindung von Faktor IX an die Rezeptoren der Endothelzellen verhindern (vgl. US-A-4885277).

Gegenstand der Erfindung ist deshalb auch die Verwendung von hochreinem rekombinantem pro-Faktor IX als Faktor IX-Antagonist sowie die Verwendung von hochreinem rekombinantem pro-Faktor IX zur Herstellung einer pharmazeutischen Zusammensetzung mit einer antagonistischen Wirkung gegenüber Faktor IX.

In den folgenden Beispielen wird die Erfindung näher erläutert, ohne sie darauf zu beschränken.

Beispiel 1 beschreibt dabei die Trennung von pro-Faktor IX von Faktor IX an einer Ionenaustauschersäule durch einen linearen Salzgradienten. Beispiel 2 beschreibt die Trennung von pro-Faktor IX von Faktor IX durch eine stufenweise Elution der Anionenaustauschersäule. Beispiel 3 beschreibt die Trennung von pro-Faktor IX und Faktor IX durch eine pH-abhängige Elution. In Beispiel 4 ist die Trennung von pro-Faktor IX und Faktor IX durch eine Immunaffinitätschromatographie beschrieben.

In den Figuren zeigen:
Figur 1 eine lineare Chromatographie von Faktor IX/pro-Faktor IX (A: Proteinabsorption; B: Antigenkonzentration, Faktor-IX Gerinnungsaktivität);
Figur 2 eine denaturierende Elektrophorese von Faktor IX/pro-Faktor IX der Reinigungsfraktionen der linearen Chromatographie;
Figur 3 eine stufenweise Chromatographie von Faktor IX/pro-Faktor IX; und
Figur 4 eine denaturierende Elektrophorese von Faktor IX/pro-Faktor IX der stufenweisen Chromatographie (A: Mischung aus Faktor-IX/pro-Faktor-IX; B: pro-Faktor-IX; C: Faktor-IX).

### Beispiele

### Beispiel 1: Trennung von rekombinantem pro-Faktor IX von rekombinantem Faktor IX durch lineare Elution

Materialien:
Säule: Mono-Q 5/5 HR, Volumen 1 ml (Pharmacia)
Instrument: Pharmacia FPLC LCC-500.
Puffer A: 50 mM Tris/20 mM Citrat, pH 7,4, 150 mM NaCl.
Puffer B: 50 mM Tris/20 mM Citrat, pH 7,4, 300 mM NaCl.

Der Anionenaustauscher wurde regeneriert und mit Puffer A äquilibriert. Anschließend wurden 5 ml der Mischung rekombinanter Faktor IX/pro-Faktor IX, die mit dem vorstehend beschriebenen Vero/Vaccinia Expressionssystem hergestellt wurde, mit einer Geschwindigkeit von 1 ml/Minute auf die Säule aufgetragen. Durch Waschen mit Puffer A mit gleicher Fließgeschwindigkeit entfernt man nicht an der Säule gebundenes Material. Dann wurde durch Mischen von Puffer A und Puffer B die Säule mittels eines linearen NaCl-Gradienten von 150 mM bis 300 mM in 50 ml Volumen eluiert. Es wurden Fraktionen von 2 ml gesammelt. Während der Chromatographie wurde die Proteinabsorption in üblicher Weise bei 280 nm verfolgt. Die Proteinkonzentration wurde mittels der Bradford Methode (M. Bradford, Anal. Biochem. 72 (1976) 248-254) und die Aktivität von Faktor IX mittels eines handelsüblichen Gerinnungstestes (Faktor IX Gerinnung, Immuno AG) bestimmt. Die Konzentration des Faktor IX-Antigens wurde mittels ELISA (Diagnostica Stago) bestimmt.

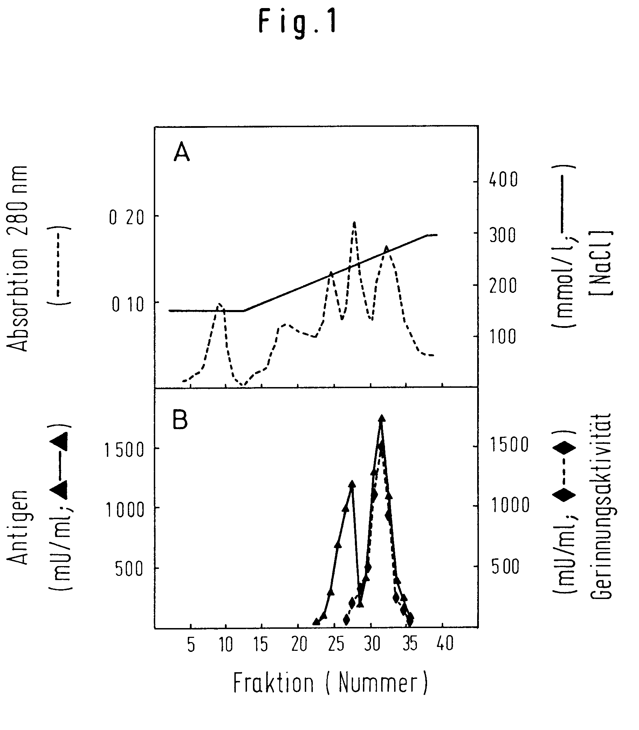

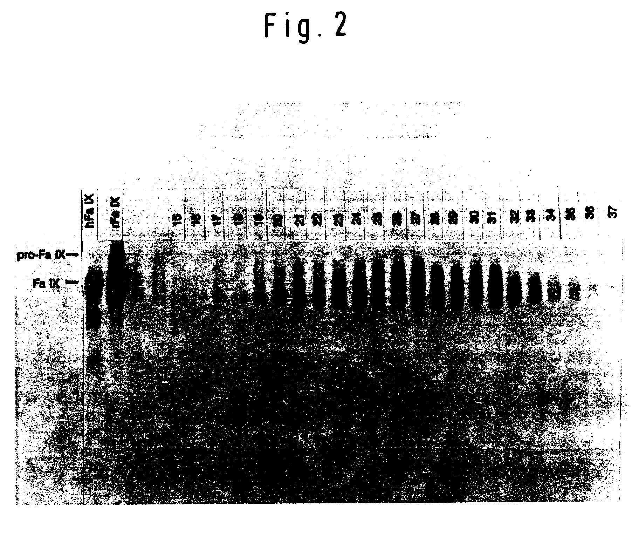

Figur 1 zeigt das gemäß Beispiel 1 erhaltene Chromatographie-Profil. Dabei ergab sich, daß Faktor IX-Antigen in zwei separaten Elutionsbereichen (Fraktion 22 bis 28 und Fraktion 29 bis 35) von der Säule eluiert wurde. Die Untersuchung mittels denaturierender Elektrophorese (U.K. Laemmli, Nature 227 (1970) 680-685) ergab, daß das Faktor IX-Antigen der Fraktionen 22 bis 28 ein um etwa 2000 höheres Molekulargewicht aufweist, als das Faktor IX-Antigen der Fraktionen 29 bis 35 (Figur 2). Die Untersuchung des Gerinnungspotentials der Elutionsfraktionen ergab, daß physiologisch aktiver Faktor IX nur in den Fraktionen 29 bis 35 erhalten war (Tabelle 1).

**Tabelle 1**

| Trennung von pro-Faktor IX und Faktor IX durch lineare Anionenaustauschchromatographie | | | | |
|---|---|---|---|---|
| Material | Volumen (ml) | F IX:Ag (U) | F IX:C (U) | F IX:Ag/F IX:C |
| Gemisch pro-F IX/F IX | 5 | 28,0 | 17,0 | 1,7 |
| Fraktion 22-28 | 14 | 7,3 | 1,0 | 7,3 |
| Fraktion 29-35 | 14 | 10,8 | 9,5 | 1,1 |

### Beispiel 2: Trennung von rekombinantem pro-Faktor IX von rekombinantem Faktor IX durch stufenweise Elution

Materialien:
Säule: Mono-Q 5/5 HR, Volumen 1 ml (Pharmacia)
Instrument: Pharmacia FPLC LCC-500
Puffer A: 50 mM Tris/20 mM Citrat, pH 8,5, 220 mM NaCl.
Puffer B: 50 mM Tris/20 mM Citrat, pH 8,5, 300 mM NaCl.

Als Ausgangsmaterial diente wie im Beispiel 1 ein Gemisch aus rekombinantem pro-Faktor IX und Faktor IX, die mit dem vorstehend beschriebenen Vero/Vaccinia Expressionssystem hergestellt wurde.

Die Ionenaustauschersäule wurde regeneriert und mit Puffer A äquilibriert. Anschließend wurden 90 ml des Faktor IX/pro-Faktor IX-Gemisches mit einer Geschwindigkeit von 1 ml/Minute auf die Säule aufgetragen. Anschließend wurde die Säule mit Puffer A gespült. An die Säule gebundenes Protein wurde dann durch Elution mit Puffer B eluiert. Es wurden Fraktionen von 2 ml gesammelt. Die Protein- bzw. die Faktor IX-Antigenkonzentration sowie die Gerinnungstests wurden wie in Beispiel 1 beschrieben durchgeführt.

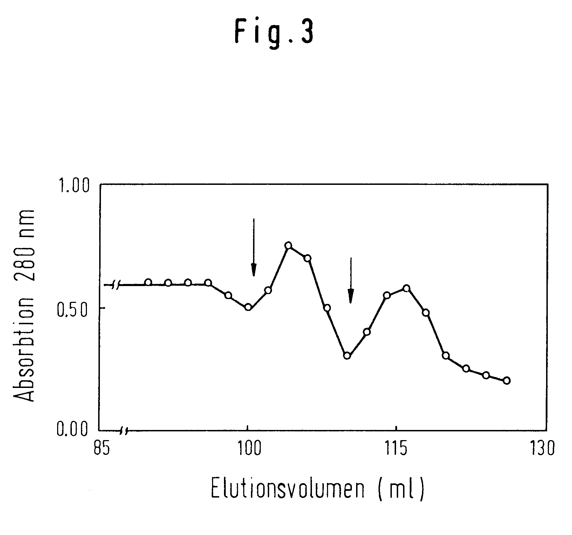

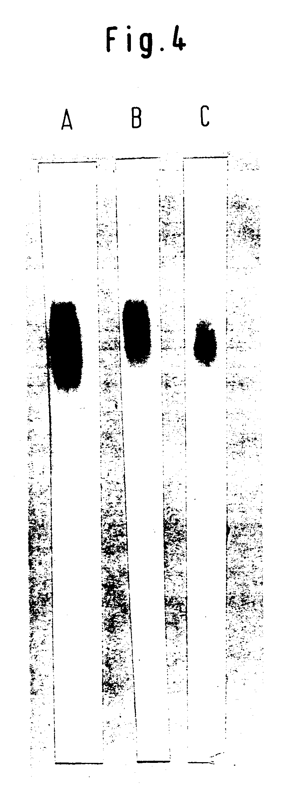

Figur 3 zeigt das gemäß Beispiel 2 erhaltene Chromatographie-Profil. Dabei ergab sich, daß Faktor IX-Antigen sowohl im Elutionspuffer A (Elutionsvolumen 100 ml bis 110 ml) als auch im Elutionspuffer B (Elutionsvolumen 111 ml bis 125 ml) bestimmt werden konnte. Gerinnungstests der Elutionsfraktionen ergaben jedoch, daß sich physiologisch aktiver Faktor IX nur im Elutionspuffer B fand (Tabelle 2). Die Untersuchung mittels denaturierender Elektrophorese (U.K. Laemmli, l.c.) ergab, daß das Faktor IX-Antigen des Elutionspuffers A ein um etwa 2000 höheres Molekulargewicht aufwies, als aktiver Faktor IX des Elutionspuffers B (Figur 4).

**Tabelle 2**

| Trennung von pro-Faktor IX von Faktor IX durch stufenweise Anionenaustauschchromatographie. | | | | |
|---|---|---|---|---|
| Material | Volumen (ml) | F IX:Ag (U) | F IX:C (U) | F IX:Ag/F IX:C |
| Gemisch pro-F IX/F IX | 90 | 81,0 | 45,0 | 1,8 |
| Puffer A | 10 | 31,0 | 1,0 | 31,0 |
| Puffer B | 10 | 45,0 | 40,0 | 1,1 |

### Beispiel 3: Trennung von rekombinantem pro-Faktor IX von rekombinantem Faktor IX durch pH-abhängige Elution.

Materialien:
Säule: Mono-Q 5/5 HR, Volumen 1 ml (Pharmacia)
Instrument: Pharmacia FPCL LCC-500
Puffer A: 20 mM Tris/HCl Puffer, pH 8,0, 150 mM NaCl.
Puffer B: 20 mM Tris/HCl Puffer, pH 6,0, 150 mM NaCl.

Eine Mischung aus rekombinantem Faktor IX und pro-Faktor IX wurde wie im Beispiel 1 beschrieben hergestellt.

Die Ionenaustauschersäule wurde regeneriert und mit Puffer A äquilibriert. Anschließend wurden 45 ml rekombinantes Faktor IX/pro-Faktor IX -Gemisch mit einer Geschwindigkeit von 1 ml/Minute auf die Säule aufgetragen. Anschließend wurde die Säule mit einem absteigenden pH-Gradienten von pH = 8,0 bis pH = 6,0 durch Mischen aus Puffer A und B eluiert. Es wurden Fraktionen von 2 ml gesammelt. Nach der Chromatographie wurde die Proteinkonzentration mittels der Bradford Methode (M. Bradford, l.c.) bestimmt. Die Aktivität von Faktor IX wurde mittels eines handelsüblichen Gerinnungstestes (Faktor IX Gerinnung, Immuno AG) bestimmt. Die Konzentration des Faktor IX-Antigens wurde mittels ELISA (Diagnostica Stago) bestimmt. Die Ergebnisse ergaben, daß Faktor IX-Antigen sowohl im Eluat bei pH = 7,0 bis pH = 7,4 als auch bei pH = 6,0 bis 6,7 bestimmt werden konnte.

Die weiteren Untersuchungen der Elutionsfraktionen ergaben jedoch, daß sich physiologisch aktiver Faktor IX nur in der Elutionsfraktion pH = 6,0 bis 6,7 enthalten war. In Tabelle 3 sind die Meßwerte der Trennung von pro-Faktor IX von Faktor IX zusammengestellt.

**Tabelle 3**

| Trennung von pro-Faktor IX von Faktor IX durch pH abhängige Anionenaustauschchromatographie | | | | |
|---|---|---|---|---|
| Material | Volumen (ml) | F IX:Ag (U) | F IX:C (U) | F IX:Ag/F IX:C |
| Gemisch pro-F IX/F IX | 45 | 65 | 40 | 1,6 |
| Eluat pH 7,0-7,4 | 10 | 28 | 4 | 7,0 |
| Eluat pH 6,0-6,7 | 12 | 33 | 30 | 1,1 |

### Beispiel 4: Trennung von rekombinantem pro-Faktor IX von rekombinantem Faktor IX durch Immunaffinitätschromatographie

Materialien:
Säule: Anti-Pro-Sequenz-Faktor IX-Sepharose, Volumen 3 ml
Instrument: Pharmacia FPCL LCC-500
Puffer A: 20 mM Tris/HCl Puffer, pH 7,4
Puffer B: 20 mM Tris/HCl Puffer, pH 7,4, 3 M KSCN
Eine Mischung aus rekombinantem Faktor IX und pro-Faktor IX wurde wie in Beispiel 1 beschrieben hergestellt.

Durch Immunisierung einer Ziege mit gereinigtem pro-Peptid wurde ein Antiserum gewonnen, das den pro-Faktor IX bindet. Dieser polyklonale Antikörper wurde entsprechend der Herstellervorschrift an Bromcyan-aktivierte Sepharose (Pharmacia) gekoppelt. Es wurde eine Glassäule mit dem Immunaffinitätsgel gefüllt und mit Puffer A equilibriert. Anschließend wurden 33 ml des Gemisches aus rekombinantem Faktor IX und pro-Faktor IX mit einer Geschwindigkeit von 1 ml/min. auf die Säule aufgetragen. Anschließend wurde die Säule mit 5 ml Puffer A gespült. An der Säule gebundenes Protein wurde dann durch Elution mit Puffer B eluiert. Es wurden Fraktionen von 2 ml gesammelt.

Nach der Chromatographie wurde die Proteinkonzentration mittels der Methode nach Bradford (l.c.) bestimmt. Die Konzentration des Faktor IX-Antigens wurde mittels ELISA (Diagnostica Stago) bestimmt. Die Aktivität von Faktor IX wurde mittels eines handelsüblichen Gerinnungstestes (Faktor IX Gerinnung, Immuno AG) ermittelt.

Die Bestimmungen ergaben, daß Faktor IX-Antigen sowohl im ungebundenen Eluat, d.h. in Puffer A, als auch im Puffer B bestimmt werden konnte. Die weiteren Untersuchungen der Elutionsfraktionen ergaben jedoch, daß sich physiologisch aktiver Faktor IX nur in der ungebundenen Fraktion (Puffer A) befand. Pro-Faktor IX wurde durch den Antikörper an die Säule gebunden und erst durch Erhöhung der Salzkonzentration mit Puffer B wieder eluiert. Tabelle 4 faßt die Ergebnisse der Trennung von pro-Faktor IX von Faktor IX zusammen.

**Tabelle 4**

| Trennung von pro-Faktor IX von Faktor IX durch Immunaffinitätschromatographie | | | | |
|---|---|---|---|---|
| Material | Volumen U | F IX:Ag U | F IX:C U | F IX:Ag/FIX:C |
| Gemisch pro-FIX/FIX | 33 | 150 | 90 | 1,6 |
| Puffer A | 35 | 91 | 80 | 1,1 |
| Puffer B | 10 | 50 | 3 | 17,0 |

## Patentansprüche

1. Verfahren zur Trennung von rekombinantem pro-Faktor IX von rekombinantem Faktor IX durch eine chromatographische Methode.

2. Verfahren nach Anspruch 1, dadurch gekennzeichnet, daß man die Mischung der beiden Proteine an einen Ionenaustauscher bindet und pro-Faktor IX und Faktor IX durch Pufferlösungen mit unterschiedlichen Salzkonzentrationen und/oder pH-Werten voneinander getrennt eluiert.

3. Verfahren nach Anspruch 2, dadurch gekennzeichnet, daß als Ionenaustauscher ein Anionenaustauscher verwendet wird.

4. Verfahren nach Anspruch 3, dadurch gekennzeichnet, daß als Anionenaustauscher ein solcher vom Typ QAE, DEAE oder TMAE verwendet wird.

5. Verfahren nach einem der Ansprüche 2 bis 4, dadurch gekennzeichnet, daß die Elution von pro-Faktor IX und Faktor IX durch einen steigenden Salzgradienten und/oder einen fallenden pH-Gradienten erfolgt.

6. Verfahren nach einem der Ansprüche 2 bis 5, dadurch gekennzeichnet, daß als Elutionspuffer Pufferlösungen mit pH-Werten zwischen 5,0 und 10,0 verwendet werden.

7. Verfahren nach einem der Ansprüche 2 bis 6, dadurch gekennzeichnet, daß als Elutionspuffer Pufferlösungen, die ein Salz eines univalenten Kations enthalten, verwendet werden.

8. Verfahren nach einem der Ansprüche 2 bis 7, dadurch gekennzeichnet, daß die Salzkonzentration des Elutionspuffers im Bereich von 10 mM bis 1000 mM liegt.

9. Verfahren nach einem der Ansprüche 2 bis 8, dadurch gekennzeichnet, daß die Elution des pro-Faktors IX bei geringeren Salzkonzentrationen oder höheren pH-Werten als die Elution des Faktors IX erfolgt.

10. Verfahren nach Anspruch 1, dadurch gekennzeichnet, daß man eines der beiden Proteine an eine chromatographische Säule bindet, das andere im Eluat abtrennt und das gebundene Protein schließlich durch Veränderung in der Pufferzusammensetzung eluiert.

11. Verfahren nach einem der Ansprüche 1 und 10, dadurch gekennzeichnet, daß das vorerst gebundene Protein mit Pufferlösungen eluiert wird, die ein anorganisches oder organisches Salz enthalten, vorzugsweise NaCl, MgCl2, KSCN oder Harnstoff, oder die ein hydrophobes Agens, vorzugsweise Ethylenglykol, enthalten.

12. Verfahren nach einem der Ansprüche 1, 10 und 11, dadurch gekennzeichnet, daß die Salzkonzentration zur Eluierung des gebundenen Proteins im Bereich von 500 mM bis 3,5 M oder die Agenskonzentration im Bereich von 0,1 - 50 Gew.-%, vorzugsweise 0,1 bis 10 Gew.-%, liegen.

13. Verfahren nach einem der Ansprüche 1 und 10, dadurch gekennzeichnet, daß das vorerst gebundene Protein durch Veränderung des pH-Wertes der Pufferlösung eluiert wird.

14. Verfahren nach einem der Ansprüche 1, 10 und 13, dadurch gekennzeichnet, daß der pH-Wert der Elutionspuffers unter 7,0, vorzugsweise unter 5,0, oder über 8,0, vorzugsweise über 9,0 liegt.

15. Verfahren nach einem der Ansprüche 1 und 10 bis 14, dadurch gekennzeichnet, daß eine Immunaffinitätssäule einen monoklonalen oder polyklonalen Antikörper, der entweder das pro-Peptid des pro-Faktors IX oder den Faktor IX erkennt, gebunden hat.

16. Verfahren nach einem der Ansprüche 1 und 10 bis 15, dadurch gekennzeichnet, daß die Immunaffinitätssäule einen monoklonalen oder polyklonalen Antikörper, der das pro-Peptid des pro-Faktors IX erkennt, gebunden hat.

17. Verfahren nach einem der Ansprüche 1 und 10 bis 16, dadurch gekennzeichnet, daß der pro-Faktor IX an die Säule gebunden wird und reiner Faktor IX im Eluat vorliegt.

18. Verfahren nach einem der Ansprüche 1 und 11 bis 17, dadurch gekennzeichnet, daß der pro-Faktor IX durch Erhöhung der Salzkonzentration, Zugabe von Agens oder Veränderung des pH-Wertes von der Säule eluiert wird.

19. Hochreiner rekombinanter Faktor IX, der frei ist von pro-Faktor IX, mindestens jedoch 95 % rein ist, erhältlich nach einem Verfahren gemäß einem der Ansprüche 1 bis 18.

20. Hochreiner rekombinanter Faktor IX nach Anspruch 19, dadurch gekennzeichnet, daß er mindestens 98 % rein ist.

21. Hochreiner rekombinanter Faktor IX nach Anspruch 19 oder 20, dadurch gekennzeichnet, daß das Verhältnis von Faktor IX-Antigen zu aktivem Faktor IX bei ≦ 1,1 liegt.

22. Hochreiner rekombinanter pro-Faktor IX, der frei ist von Faktor IX, mindestens jedoch 95 % rein ist, erhältlich nach einem Verfahren gemäß einem der Ansprüche 1 bis 18.

23. Hochreiner rekombinanter pro-Faktor IX nach Anspruch 22, dadurch gekennzeichnet, daß er mindestens 98 % rein ist.

24. Pharmazeutische Zusammensetzung, dadurch gekennzeichnet, daß sie einen hochreinen Faktor IX nach einem der Ansprüche 19 bis 21 in einem physiologisch annehmbaren Träger enthält.

25. Pharmazeutische Zusammensetzung, dadurch gekennzeichnet, daß sie einen hochreinen pro-Faktor IX nach Anspruch 22 oder 23 in einem physiologisch annehmbaren Träger enthält.

26. Verwendung von hochreinem rekombinantem pro-Faktor IX als Faktor IX-Antagonist.

27. Verwendung von hochreinem rekombinantem pro-Faktor IX zur Herstellung einer pharmazeutischen Zusammensetzung mit einer antagonistischen Wirkung gegenüber Faktor IX.
